# EUROPÄISCHE PATENTSCHRIFT

(11) **EP 1 351 653 B1**
(45) Veröffentlichungstag und Bekanntmachung des Hinweises auf die Patenterteilung: **02.07.2008**
(21) Anmeldenummer: 02718015.7
(22) Anmeldetag: 10.01.2002
(51) Int. Cl.: A61K 8/06, A61K 8/34, A61K 8/92, A61Q 19/00

(54) **EMULSIONEN AUF BASIS SPEZIELLER EMULGATOREN**
EMULSIONS BASED ON SPECIAL EMULSIFIERS
EMULSIONS A BASE D'EMULSIFIANTS SPECIAUX

(30) Priorität: 19.01.2001 DE 10102500
(43) Veröffentlichungstag der Anmeldung: 15.10.2003
(73) Patentinhaber: Cognis IP Management GmbH, 40589 Düsseldorf (DE)
(72) Erfinder: BAUMÖLLER, Guido, 42799 Leichlingen (DE); KAWA, Rolf, 40789 Monheim (DE); EICHHORN, Stephan, 64579 Gernsheim (DE)
(86) Internationale Anmeldenummer: PCT/EP2002/000168
(87) Internationale Veröffentlichungsnummer: WO 2002/056842

(56) Entgegenhaltungen:
- EP-A- 1 029 977
- DE-A- 19 815 086
- DE-A- 19 821 402
- DE-A- 19 827 661

## Beschreibung

Gegenstand der Erfindung sind spezielle, sehr milde Emulsionen, die als Körperpflegemittel sowie insbesondere zur Imprägnierung und Benetzung von Gebrauchs- und Hygienepapiertücher eingesetzt werden können.

### Stand der Technik

Unter dem Oberbegriff "Papier" werden ca. 3000 verschiedene Sorten und Artikel verstanden, deren Beschaffenheit und Anwendungsgebiete sich zum Teil erheblich unterscheiden können. Zur Herstellung von Papier benötigt man eine Reihe von Zusatzstoffen, von denen Füllstoffe (z.B. Kreide oder Kaolin) und Bindemittel (z.B. Stärke) zu den wichtigsten zählen. Für den Bereich der Tissue- und Hygienepapiere, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird. Im Hinblick auf die Wirtschaftlichkeit der Papierherstellung sowie aus ökologischer Sicht, ist es jedoch wünschenswert, möglichst hohe Anteile an qualitativ minderwertigerem Altpapier mitzuverwenden. Dies hat jedoch zur Folge, daß der Weichgriff des Papiers signifikant verschlechtert wird, was von den Anwendern als störend empfunden wird und insbesondere bei häufigem Gebrauch auch zu Hautirritationen führen kann.

In der Vergangenheit hat es daher nicht an Versuchen gemangelt, Tissuepapiere durch Tränken, Beschichten oder andere Oberflächenbehandlung so zu modifizieren, daß ein angenehmerer Weichgriff resultiert. Hierfür müssen spezielle Lotionen und Emulsionen entwickelt werde, die sich einerseits leicht auf das Papier aufgetragen lassen, anderseits die Papierstruktur nicht negativ beeinflussen. Um den Weichgriff zu verbessern, werden häufig Niotenside oder eine Kombination aus Nio- und Aniontensiden verwendet. Auch Polysiloxane und kationische Polymere werden für diesen Zweck eingesetzt.

Gegenstand der internationalen Patentanmeldung WO 95/35411 sind Tissuepapiere, die mit Avivagemitteln beschichtet werden, welche 20 bis 80 Gew.-% eines wasserfreien Emolliens (Mineralöle, Fettsäureester, Fettalkoholethoxylate, Fettsäureethoxylate, Fettalkohole und deren Mischungen), 5 bis 95 Gew.-% eines das Emolliens "immobilisierenden Agens" (Fettalkohole, Fettsäuren oder Fettalkoholethoxylate mit jeweils 12 bis 22 Kohlenstoffatomen im Fettrest) sowie 1 bis 50 Gew.-% Tenside mit einem HLB-Wert von vorzugsweise 4 bis 20 enthalten. Die in der Schrift aufgeführten Ausführungsbeispiele enthalten als Emolliens ausnahmslos Petrolatum. Die internationale Patentanmeldung WO 95/35412 offenbart ähnliche Tissuepapiere, wobei als Softener wasserfreie Mischungen von (a) Mineralölen, (b) Fettalkoholen oder Fettsäuren und (c) Fettalkoholethoxylaten zum Einsatz kommen. Gegenstand der intenationalen Patentanmeldung WO 95/16824 sind Avivagemittel für Tissuepapiere, die Mineralöl, Fettalkoholethoxylate und nichtionische Tenside (Sorbitanester, Glucamide) enthalten. Des weiteren werden in der internationalen Patentanmeldung WO 97/30216 (Kaysersberg) flüssige Avivagemittel für Papiertaschentücher auf Basis von langkettigen, gesättigten Fettalkoholen und Wachsestem mit insgesamt wenigstens 24 Kohlenstoffatomen beschrieben, die einen sehr hohen Wasseranteil enthalten. Vom anwendungstechnischen Standpunkt sind Weichgriff, Verarbeitungsverhalten und Sensorik der behandelten Papiere nach wie vor verbesserungswürdig.

Der Erfindung lag die Aufgabe zugrunde, Emulsionen zur Verfügung zu stellen, mit deren Hilfe man trockene Gebrauchspapiere, insbesondere Tissuepapiere, aber auch Tissuegewebe mit besonders angenehmen Weichgriff herstellen kann. Die Emulsionen sollten ausgezeichnete pflegende Eigenschaften aufweisen, hinsichtlich sensorischer Eigenschaften denen klassischer Skin-Care-Formulierungen ähneln und sich durch besondere Milde und Hautverträglichkeit auszeichnen. Ein weiterer Aspekt der Aufgabe bestand darin, Zubereitungen zur Verfügung zu stellen, die auch mit Tissue-Papieren mit einem hohen Altpapieranteil kompatibel sind. Gleichzeitig sollten nur leicht biologisch abbaubare Hilfsstoffe Verwendung finden und die Zubereitungen leicht in das Tissue eindringen, sich homogen verteilen, einen relativ niedrigen Wassergehalt aufweisen und dennoch in hochkonzentrierter Form eine so niedrige Viskosität aufweisen, daß sie sich leicht verarbeiten lassen.

### Beschreibung der Erfindung

Es wurde nun überraschenderweise gefunden, daß Zubereitungen auf Basis bestimmter Emulgatoren und Ölkörper sowie einem definierten Wassergehalt den Weichgriff der Papierprodukte signifikant verbessern, ausgezeichnete sensorische Eigenschaften aufweisen, auch bei besonders kritischem Tissue-Papier mit einem hohen Altpapieranteil leicht verarbeitbar sind und sich durch besondere Milde auszeichnen.

Gegenstand der Erfindung sind daher W/O- und O/W-Emulsionen enthaltend:
(a) Polyolpoly-12-hydroxystearate
(b) Ölkörper ausgewählt aus der Gruppe der Dialkylether, Dialkylcarbonate, der Glyceride, der Kohlenwasserstoffe und der Silikonöle oder beliebigen Gemischen davon
(c) 5-25 Gew.-% Wasser
dadurch gekennzeichnet, dass das Gewichtsverhältnis der Komponenten (a : b) 0,2 - 2,0 beträgt.

Die erfindungsgemäßen Emulsionen sind auch in hochkonzentrierter Form niedrigviskos, so daß sie sich leicht verarbeiten lassen. In einer bevorzugten Ausführungsform weisen die Emulsionen bei 23° C eine Viskosität von 100 -10000 mPa·s auf, wobei ein Bereich von 500 - 5000 mPa·s und insbesondere von 2000 - 4000 mPa·s bevorzugt ist (Brookfield-RVF, Spindel 5, 10 UpM, 23° C). Die Emulsionen enthalten neben speziellen Emulgatoren und ausgewählten Ölkörpem 5 - 25 Gew.-%, vorzugsweise 10 - 25 Gew.-% und insbesondere 12 - 20 Gew.-% Wasser. Vorzugsweise liegen die erfindungsgemäßen Zusammensetzungen als W/O-Emulsionen vor. Infolge der geringen Tröpfchengröße dringen die Emulsionen sehr rasch in die Tissues ein und verteilen sich homogen. Ein weiterer Vorteil besteht ferner darin, daß die praktisch geruchsfreien Zubereitungen ökotoxikologisch unbedenklich sind und insbesondere leicht biologisch abgebaut werden können. Die erfindungsgemäßen Emulsionen eignen sich insbesondere auch als sehr milde Körperpflegemittel.

### Polyolpolyr-12-hydroxystearate

Bei den Polyolpoly-12-hydroxystearaten, welche die Komponente (a) bilden, handelt es sich um bekannte Stoffe, die beispielsweise unter den Marken "Dehymuls^{®} PGPH" oder "Eumulgin^{®} VL 75" (Abmischung mit Coco Glucosides im Gewichtsverhältnis 1:1) oder Dehymuls^{®} SBL von der Cognis Deutschland GmbH vertrieben werden. In diesem Zusammenhang sei insbesondere auf das Europäische PatentEP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methyfolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit;
(f) Zucker mit 5 bis 12 Kohlenstoffatomen, wie beispielsweise Glucose oder Saccharose;
(g) Aminozucker, wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft haben sich Umsetzungsprodukte von Poly-12-hydroxystearinsäure mit Polyglycerinen folgender Homologenverteilung erwiesen (bevorzugte Mengen sind in Klammem angegeben):

| | |
|---|---|
| Glycerine | : 5 bis 35 (15 bis 30) Gew.-% |
| Diglycerine | : 15 bis 40 (20 bis 32) Gew.-% |
| Triglycerine | : 10 bis 35 (15 bis 25) Gew.-% |
| Tetraglycerine | : 5 bis 20 (8 bis 15) Gew.-% |
| Pentaglycerine | : 2 bis 10 (3 bis 8) Gew.-% |
| Oligoglycerine | : ad 100 Gew.-% |

Erfindungsgemäß besonders vorteilhaft ist der Einsatz von Dehymuls^{®} SBL, einem von der Cognis Deutschland GmbH vertriebenen Emulgatorgemisch auf Basis von Polyglycerinpoly-12-hydroxystearat sowie Dicaprylether und Cocoglyceriden als Ölkörper (Gewichtsverhältnis 1:1:1).

Die Polyolpoly-12-hydroxystearate sind in den erfindungsgemäßen Emulsionen in einer Menge 3 - 30 Gew.-%, vorzugsweise 10 - 25 Gew.-% und besonders bevorzugt 15 - 25 Gew.-% enthalten.

### Ölkörper

Unter den Ölkörpern, die als Komponente (b) Verwendung finden, sind erfindungsgemäß Stoffe zu verstehen, die aus der Gruppe der Glyceride, Kohlenwasserstoffe, Silikonöle, Dialkylether und der Dialkylcarbonate oder beliebigen Gemischen davon ausgewählt sind, wobei die Gesamtheit der Ölkörper bei 20° C vorzugsweise flüssig ist. Erfindungsgemäß besonders bevorzugt ist es, dünnflüssige Ölkörper einzusetzen, die eine Viskosität im Bereich von 1-100 mPa·s und vorzugsweise 1 - 50 mPa·s aufweisen (Höppler/Kugelfall-Methode, Deutsche Gesellschaft für Fettchemie, DGF C-IV 7,20° C). Die Ölkörper sind in den erfindungsgemäßen Emulsionen üblicherweise in einer Menge von 20 - 70 Gew.-% bezogen auf die Gesamtzusammensetzung enthalten. Mengen von 20 - 60 Gew.-% und insbesondere 30 - 50 Gew.-% sind bevorzugt.

Glyceride sind Fettsäureester des Glycerins, die natürlicher (tierischer und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Als Fettsäure sind erfindungsgemäß C₆-C₂₄-Fettsäuren, und unter diesen C₆-C₁₈-Fettsäuren, und insbesondere C₈-C₁₈-Fettsäuren bevorzugt geeignet. Die Fettsäuren können verzweigt oder unverzweigt, gesättigt oder ungesättigt sein. Erfindungsgemäß bevorzugt ist die Verwendung bei 20 °C flüssiger Glyceride pflanzlicher Herkunft, insbesondere von Cocoglyceriden, einer Mischung aus vorwiegend Di- und Triglyceriden mit C₈-C₁₈-Fettsäuren, die beispielsweise unter der Bezeichnung Myritol^{®} 331 von der Cognis Deutschland GmbH vertrieben werden.

Zu den erfindungsgemäß einsetzbaren Ölkörpem zählen auch natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkylcyclohexane. Um eine gute Verteilbarkeit der Zusammensetzung auf dem Papier zu gewährleisten, sind bei 20 °C flüssige Kohlenwasserstoffe bevorzugt geeignet.

Erfindungsgemäß sind als Ölkörper auch flüssige Siliconöle geeignet. Zu diesen zählen z. B. Dialkyl- und Alkylarylsiloxane, wie beispielsweise Cyclomethicone, Dimethylpolysiloxan und Methylphenylpolysiloxan, sowie deren alkoxylierte und quaternierte Analoga. Geeignete nicht-flüchtige Siliconöle, wie z. B. Polyalkylsiloxane, Polyalkylarylsiloxane und Polyethersiloxan-Copolymere sind in Cosmetics: Science and Technology, Hrsg.: M. Balsam und E. Sagarin, Bd. 1, 1972, S. 27-104, in US 4,202,879 und US 5,069,897 beschrieben.

Erfindungsgemäß geeignet als Ölkörper sind auch lineare oder verzweigte, symmetrische oder unsymmetrische, gesättigte oder ungesättigte Di-n-alkyl(en)ether mit 12 - 24 C-Atomen pro Alkyl(en)gruppe, wie z. B. Di-n-octylether, Di-(2-ethylhexyl)-ether, Laurylmethylether oder Octylbutylether, Didodecylether oder Dibehenylether, wobei bei 20 °C flüssige Ether als Ölkomponenten bevorzugt sind.

Ebenso sind lineare oder verzweigte, gesättigte oder ungesättigte C₆-C₂₂-Fettalkoholcarbonate geeignet. Diese Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996**)**. Typische Beispiele für Dialkyl(en)carbonate sind vollständige oder partielle Umesterungsprodukte von Dimethyl- und/oder Diethylcarbonat mit Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachidylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen anfallen. Bevorzugt geeignet sind bei 20 °C dünnflüssige Dialkyl(en)carbonate, wie beispielsweise Dihexyl-, Dioctyl-, Di-(2-ethylhexyl)- oder Dioleylcarbonat.

Eine bevorzugte Ausführungsform der Emulsion enthält als Ölkörper Dicaprylether, C₈-C₁₈-Glyceride oder ein Gemisch dieser Substanzen.

### Feuchthaltemittel/Hautbefeuchtungsmittel

In einer weiteren bevorzugten Ausführungsform enthält die erfindungsgemäße Emulsion wenigstens ein Feuchthaltemittel, das zur Verbesserung der sensorischen Eigenschaften und Stabilität der Zusammensetzung beiträgt, der Feuchtigkeitsregulierung der Haut dient und das Eindringvermögen der Emulsion auf Papier fördert. Die Feuchthaltemittel sind üblicherweise in einer Menge von 1- 20 Gew.%, vorzugsweise 5 -15 Gew.%, und insbesondere 5 -10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansatzei-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin und Triglycerin.

### Reizlindernde/entzündungshemmende Wirkstoffe

Eine bevorzugte Ausführungsform der erfindungsgemäßen Emulsion enthält wenigstens einen reizlindernden/entzündunsghemmenden Wirkstoff, der insbesondere zur Linderung entzündlicher Hautprozesse oder geröteter, wunder Haut dient. Der reizlindemde Wirkstoff ist üblicherweise in einer Menge von 0,01-10 Gew.-%, vorzugsweise 0,1- 7 Gew.-% und insbesondere 1 - 5 Gew.-% enthalten.

Erfindungsgemäß bevorzugt sind insbesondere Bisabolol, Allantoin und Panthenol und Bisabolol. Auch -Vitamine und Vitaminvorstufen sowie Proteinhydrolysate können die Wundheilung fördern.

Geeignet sind auch Pflanzenextrakte, die häufig eine synergistisch wirkende Kombination wundheilender/reizlindernder Stoffe enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf **Seite 44 der 3. Auflage des Leitfadens zur Inhaltsstoffdeklaration kosmetischer Mittel, herausgegeben vom Industrieverband Körperpflege- und Waschmittel e.V. (lKW), Frankfurt**, beginnenden Tabelle aufgeführt sind.

Erfindungsgemäß sind vor allem die Extrakte aus Kamille, Aloe Vera, Hamamelis, Lindenblüten, Roßkastanie, Grünem Tee, Eichenrinde, Brennessel, Hopfen, Klettenwurzel, Schachtelhalm, Weißdorn, Mandel, Fichtennadel, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel geeignet.

Als Extraktionsmittel zur Herstellung der genannten Pflanzenextrakte können-Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

### Konsistenzgeber

Als Konsistenzgeber werden Stoffe bezeichnet, die in Emulsionen eine Verdickung, also Viskositätserhöhung bewirken. Die Konsistenzgeber sind üblicherweise in einer Menge von 0,0 -10 Gew.-%, vorzugsweise 0,5 - 8 Gew.% und insbesondere 2 - 7 Gew.-% enthalten. Erfindungsgemäß eignen sich insbesondere Fettalkohole, Wachse und Seifen, die bei 20° C eine feste Konsistenz aufweisen.

Bevorzugt einsetzbar im Sinne der Erfindung sind bei 20 °C feste C₁₂-C₂₄-Fettalkohole, die vorzugsweise gesättigt sind. Hierzu zählen beispielsweise Myristylalkohol, Cetylalkohol, Stearyfalkohol, Erucylakohol, Ricinolalkohol, Arachidylalkohol, Behenylalkohol, Brassidylalkohole sowie deren Guerbet-Alkohole. Erfindungsgemäß einsetzbar sind auch Fettalkoholschnitte, die durch Reduktion natürlich vorkommender Fette und Öle, wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett entstehen. Es können aber auch synthetische Alkohole, z. B. die linearen, geradzahligen Fettalkohole der Ziegler-Synthese (Alfole^{®}) oder die teilweise verzweigten Alkohole aus der Oxosynthese (Dobanole^{®}) verwendet werden. Erfindungsgemäß besonders gut geeignet ist eine Mischung aus C₁₆/C₁₈-Fettalkoholen, die von der Cognis Deutschland GmbH unter der Bezeichnung Lanette^{®} O vermarktet wird.

Als Konsistenzgeber eignen sich erfindungsgemäß auch bei 20° C feste Mono-, Di- und Triglyceride sowie entsprechende Gemische auf Basis von linearen C₁₂-C₂₂-Fettsäuren oder linearen C₁₂-C₂₂-Hydroxyfettsäuren.

Unter Wachsen werden natürliche oder künstlich gewonnene Stoffe mit folgenden Eigenschaften verstanden: sie sind von fester bis brüchig harter Konsistenz, grob bis feinkristallin, durchscheinend bis opak, jedoch nicht glasartig, und schmelzen oberhalb von 35° C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß verwendbar sind natürliche pflanzliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse oder Ester langkettiger Carbonsäuren mit langkettigen Fettalkoholen. Erfindungsgemäß bevorzugt ist der Einsatz von Bienenwachs als Konsistenzgeber.

Unter den Seifen werden erfindungsgemäß Salze von Fettsäuren verstanden. Bevorzugte Konsistenzgeber sind Alkalimetall- und Erdalkalimetall- sowie Aluminiumsalze von C₁₂-C₂₄-Fettsäuren oder C₁₂-C₂₄-Hydroxyfettsäuren, wobei Calcium-, Magnesium- oder Aluminiumstearat bevorzugt ist. Besonders bevorzugt als Konsistenzgeber ist eine Kombination aus Bienenwachs und Aluminiumstearat im Gewichtsverhältnis 1:1, die in den erfindungsgemäßen Zusammensetzungen vorzugsweise in einer Menge von 1 -10 und insbesondere 3-8 Gew.-% bezogen auf die Gesamtzusammensetzung enthalten ist.

### Coemulgatoren

Eine weitere bevorzugte Ausführungsform der erfindungsgemäßen Emulsion enthält zusätzlich wenigstens einen weiteren Emulgator ausgewählt aus der Gruppe der Niotenside. Nicht-ionische Emulgatoren zeichnen sich durch ihre Hautfreundlichkeit und Milde sowie ihre ökotoxologisch guten Eigenschaften aus. Durch Verwendung einer Kombination nicht-ionischer Emulgatoren erhält man besonders feinteilige Emulsionen, so daß die Stabilität der Zusammensetzung erhöht wird. Die erfindungsgemäß Zusammensetzung enthält die Coemulgatoren in einer Menge von 0 bis 15 Gew.-%, vorzugsweise 1 bis 10 Gew.-% und insbesondere 3 bis 10 Gew.-% bezogen auf das Gesamtgewicht der Zusammensetzung.

### Nicht-ionische Emulgatoren

Zur Gruppe der nicht-ionischen Emulgatoren gehören:
(1) Anlagerungsprodukte von 2 bis 50 Mol Ethylenoxid und/oder 0 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C_{12/18}-Fettsäuremono- und -diester von Anlagerungsprodukten von 1 bis 50 Mol Ethylenoxid an Glycerin.
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Andere Polyol- und insbesondere Polyglycerinester als Polyolpoly-12-hydroxystearate, wie z. B. Polyglycerinpolyricinoleat, oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose).
(9) Wollwachsalkohole.
(10) Polysiloxan-Polyalkyl-Polyether-Copolymere bzw. entsprechende Derivate.
(11) Mischester aus Pentaerythrit, Fettsäuren, Citronensäure und Fettalkohol gemäß und/oder Mischester von Fettsäuren mit 6 bis 22 Kohlenstoffatomen, Methylglucose und Polyolen, vorzugsweise Glycerin oder Polyglycerin.
(12) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 20 24 051 als Rückfettungsmittel für kosmetische Zubereitungen bekannt.

Da W/O-Emulsionen eine bevorzugte Ausführungsform darstellen, ist es besonders bevorzugt, nicht-ionische Coemulgatoren aus der Gruppe der lipophilen W/O-Emutgatoren einzusetzen. Als lipophile Coemulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte läßt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₃-C₆-Polyolen, wie beispielsweise Glycerylmonoestem, Partialester des Pentaerythrits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Coemulgatoren sind auch Anlagerungsprodukte von 1 bis 30, vorzugsweise 5 bis 10 Mol Ethylenoxid an die genannten Sorbitanester geeignet. Eine erfindungsgemäß bevorzugte Emulgatorkombination ist ein Gemisch aus Polyglycerinpoly-12-hydroxystearat (Dehymuls^{®} PGPH), Sorbitansesquioleat (Dehymuls^{®} SSO) und Dicocoylpentaerythrityldistearylcitrat (Dehymuls^{®} FCE).

Emulsionen, die zusätzlich wenigstens ein Alkyloligoglycosid als nicht-ionischen Coemulgator enthalten, sind erfindungsgemäß bevorzugt. C₈-C₁₈-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet.

Eine besonders bevorzugte Ausführungsform der erfindungsgemäßen Zusammensetzung enthält
(a) 3 bis 25 Gew.-% Polyglycerinpoly-12-hydroxystearat; (b) 20 bis 70 Gew.-% dünnflüssiger Ölkörper ausgewählt aus der Gruppe der C₈-C₁₈-Glyceride, Dicaprylether oder einem beliebigen Gemisch davon;
(c) 0 bis 25 Gew.-% Alkyloligoglucosid; (d) 2 bis 10 Gew.-% wenigstens eines Feuchthaltemittels und
(e) 10 bis 25 Gew.-% Wasser.

Im Falle der Ausführungsform einer O/W-Emulsion ist es weiterhin vorteilhaft, zusätzlich nicht-ionische O/W-Emulgatoren mit einem HLB-Wert von 8 - 18 einzusetzen.

### Weitere Tenside/Emulgatoren (fakultativ)

Insbesondere im Falle einer O/W-Emulsion können die Zusammensetzungen weiterhin zwitterionische, amphotere, kationische und ferner anionische Tenside enthalten.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-amino-propyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quatemisierten Proteinhydrolysate dar.

### Weitere fakultative Hilfs- und Zusatzstoffe

In einer besonderen Ausführungsform der Erfindung können die Emulsionen weitere Hilfs- und Zusatzstoffe, wie beispielsweise Überfettungsmittel, Verdickungsmittel, Polymere, Wachse, biogene Wirkstoffe, Deowirkstoffe, Filmbildner, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole^{®} von Goodrich oder Synthalene^{®} von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, **zwitterionische, amphotere und nichtionische Polymere** kommen beispielsweise VinylacetatlCrotonsäure-Copolymere, VinylpyrrolidonNinylacrylat-Copolymere, VinylacetatlButylmaleatl Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, VinylpyrrolidonNinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, α-Hydroxycarbonsäuren, Aminosäuren, Ceramide, Pseudoceramide, essentielle Öle, Pflanzenextrakte und Vitaminkomplexe zu verstehen.

Als **Deowirkstoffe** kommen z. B. Antitranspirantien wie etwa Aluminiumchlorhydrate, Aluminium-Zirkonium-Chlorohydrate sowie Zinksalze in Frage. Diese werden zur Herstellung von schweißhemmenden und desodorierenden Zubereitungen eingesetzt und wirken wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron^{®} der Clariant GmbH, befindet beispielsweise sich ein Aluminiumchlorhydrat im Handel, das der Formel [Al₂(OH)₅Cl]·2,5 H₂O entspricht und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal^{®} 36G vermarktet werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Emulsionen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan^{®} von der Ciba-Geigy, Basel/CH vertrieben wird.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
- 3-Benzylidencampher bzw. 3-Benzylidennorcampher und dessen Derivate, z. B. 3-(4-Methyl-benzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon;
- Propan-1,3-dione, wie z.B. 1-(4-tert.Butylphenyl)-3-(4'methoxyphenyl)propan-1,3-dion;
- Ketotricyclo(5.2.1.0)decan-Derivate;

Als wasserlösliche UV-Filter-Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z. B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder EusoleX^{®}T 2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt, z. B. mikronisiertes Zinkoxid.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Einige dieser Verbindungen wurden bereits unter den Feuchthaltemitteln erwähnt. Typische Beispiele sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Formulierungsbeispiele

Zur Prüfung der anwendungstechnischen Eigenschaften wurde die Stabilität der erfindungsgemäßen Emulsionen überprüft und die Sensorik in Probandentest beurteilt. Handelsübliche dreilagige Tissuepapiere mit einem Anteil an Recyclepapier von 80 % und einem Gewicht von 40 g/m² wurden mit den erfindungsgemäßen Emulsionen 1 bis 5 sowie den Vergleichszubereitungen V1 und V2 in Mengen von jeweils 2,5 g/m² behandelt. Der Weichgriff wurde dann von einem Panel bestehend aus 6 erfahrenen Testpersonen auf einer Skala von sehr weich (+++), weich (++), bis hart (+) beurteilt. Ebenso wurde das sensorische Gefühl beim Betasten der Tücher beurteilt, sowie beim Auftragen der Emulsion auf den Handrücken (Sehr gut: +++, gut: ++, befriedigend: +). Zusätzlich wurde die Stabilität der Emulsionen nach 12-wöchiger Lagerung bei 23° C beurteilt (stabil: +++, weniger stabil: ++, instabil: +).

Die Ergebnisse, die Mittelwerte von drei Versuchsreihen darstellen, sind in nachfolgender Tabelle wiedergegeben.

Die Mengenangaben in nachfolgenden Beispielen beziehen sich, soweit nicht anders angegeben, auf Gew.-% Aktivsubstanz der Gesamtzusammensetzung. Die Beispiele 1 bis 5 sind erfindungsgemäße Formulierungen, V1 und V2 stellen Vergleichsbeispiele dar.

| **Zusammensetzung / Performance** | **1** | **2** | **3** | **4** | **5** | **V1** | **V2** |
|---|---|---|---|---|---|---|---|
| Polyglycerinpoly-12-hydroxystearate | 20,4 | 20,4 | 15,5 | 20,4 | 20,4 | - | 10,0 |
| Polyglyceryl-3 Diisostearate | - | - | - | - | - | 20,4 | 10,4 |
| Dicaprylylether | 20,4 | 20,4 | - | 15,0 | 15,4 | 25,4 | - |
| Cocoglyceride | 20,4, | 20,4 | 20,4 | - | 25,4 | 15,4 | - |
| Oleyl Erucate | - | - | 20,4 | - | - | - | 20,4 |
| Decyl Oleate | - | - | - | 25,8 | - | - | 20,4 |
| Sorbitan Sesquioleat | 4,8 | 4,8 | 9,8 | 4,8 | 4,8 | 4,8 | 4,8 |
| C₁₆-C₁₈-Alkylpolyglucosid | - | 2,5 | 1,0 | - | - | - | 2,5 |
| C₁₆-C₁₈-Fettalkohol | - | 2,5 | 1,0 | - | - | - | 2,5 |
| Cera Alba | 3,4 | 3,4 | 3,4 | 3,4 | 3,4 | 3,4 | 3,4 |
| Aluminiumsstearat | 3,4 | 3,4 | 3,4 | 3,4 | - | 3,4 | 3,4 |
| Dicocoyl Pentaerythrityl Distearyl Citrate | 2,0 | 2,0 | 2,0 | 2,0 | 2,0 | 2,0 | - |
| Bisabolol | 1,5 | 1,8 | 1,5 | 1,5 | 1,5 | 1,5 | 1,5 |
| Glycerin | 7,0 | 5,0 | 5,0 | 7,0 | 7,0 | 7,0 | - |
| Parfüm | 0,5 | 0,5 | 0,5 | 0,5 | 0,5 | 0,5 | 0,5 |
| Methylchloroisothiazolinel Methylisothiazolinone-Gemisch (3:1) | 0,1 | 0,2 | 0,2 | 0,2 | 0,2 | 0,2 | 0,2 |
| Wasser | ad 100 | | | | | | |
| ***Weichgriff*** | +++ | +++ | +++ | +++ | +++ | ++ | + |
| ***Sensorische Beurteilung*** | +++ | +++ | ++ | ++ | +++ | ++ | + |
| ***Stabilität*** | +++ | +++ | +++ | +++ | ++ | + | ++ |

## Patentansprüche

1. W/O oder O/W-Emulsion enthaltend:
(a) Polyolpoly-12-hydroxystearate
(b) Ölkörper ausgewählt aus der Gruppe der Glyceride, Kohlenwasserstoffe, Silikonöle, Dialkylether und der Dialkylcarbonate, oder beliebigen Gemischen davon
(c) 5 - 25 Gew.-% Wasser.
**dadurch gekennzeichnet, dass** das Gewichtsverhältnis der Komponenten (a : b) 0,2 - 2,0 beträgt.

2. Emulsion gemäß Anspruch 1, **dadurch gekennzeichnet, dass** als Komponente (b) ein Dicaprylether, C₈-C₁₈-Glycefide oder ein Gemisch dieser Substanzen enthalten ist.

3. Emulsion gemäß einem der Ansprüche 1 bis 2, **dadurch gekennzeichnet, dass** zusätzlich wenigstens ein Feuchthaltemittel enthalten ist.

4. Emulsion gemäß einem der Ansprüche 1 bis 3, **dadurch gekennzeichnet, dass** zusätzlich wenigstens ein reizlindernder Wirkstoff enthalten ist.

5. Emulsion gemäß einem der Ansprüche 1 bis 4, **dadurch gekennzeichnet, dass** zusätzlich wenigstens ein Konsistenzgeber enthalten ist.

6. Emulsion gemäß einem der Ansprüche 1 bis 5, **dadurch gekennzeichnet, dass** zusätzlich wenigstens ein Coemulgator ausgewählt aus der Gruppe der Niotenside enthalten ist.

7. Emulsion gemäß einem der Ansprüche 1. bis 6, **dadurch gekennzeichnet, dass** wenigstens ein Alkyloligoglycosid als Coemulgator enthalten ist.

8. Emulsion gemäß einem der Ansprüche 1 bis 7, **dadurch gekennzeichnet, dass** sie
(a) 3 - 25 Gew.-% Polyglycerinpoly-12-hydroxystearat
(b) 20 - 70 Gew.-% dünnflüssiger Ölkörper ausgewählt aus der Gruppe der C₈-C₁₈-Glyceride, Dicaprylether oder einem beliebigen Gemisch davon
(c) 0 - 25 Gew.-% Alkyloligoglucosid
(d) 2 - 10 Gew.-% wenigstens eines Feuchthaltemittels und
(e) 10 - 25 Gew.-% Wasser enthält.

9. Emulsionen gemäß einem der Ansprüche 1 bis 8 , **dadurch gekennzeichnet, dass** sie eine Viskosität von 100 - 10000 mPa·s bei 23°C aufweist.

10. Verwendung einer Emulsion gemäß einem der Ansprüche 1 bis 9 als Körperpflegemittel.

## Claims

1. W/O or O/W emulsion containing
(a) polyol poly-12-hydroxystearates
(b) oil components selected from the group of glycerides, hydrocarbons, silicone oils, dialkyl ethers and dialkyl carbonates or mixtures thereof
(c) 5 to 25% by weight of water,
**characterised in that** the ratio by weight of component (a : b) is 0.2 to 2.0.

2. Emulsion as claimed in claim 1, **characterized in that** a dicapryl ether, C₈₋₁₈ glycerides or a mixture of these substances is present as component (b).

3. Emulsion as claimed in any of claims 1 to 2, **characterized in that** at least one humectant is additionally present.

4. Emulsion as claimed in any of claims 1 to 3, **characterized in that** at least one irritation-soothing agent is additionally present.

5. Emulsion as claimed in any of claims 1 to 4, **characterized in that** at least one consistency factor is additionally present.

6. Emulsion as claimed in any of claims 1 to 5, **characterized in that** at least one co-emulsifier selected from the group of nonionic surfactants is additionally present.

7. Emulsion as claimed in any of claims 1 to 6, **characterized in that** at least one alkyl oligoglycoside is present as the co-emulsifier.

8. Emulsion as claimed in any of claims 1 to 7, **characterized in that** it contains
(a) 3 to 25% by weight of polyglycerol poly-12-hydroxystearate;
(b) 20 to 70% by weight of thinly liquid oil components selected from the group of C₈₋₁₈ glycerides, dicapryl ether or a mixture thereof;
(c) 0 to 25% by weight of alkyl oligoglucoside;
(d) 2 to 10% by weight of at least one humectant and
(e) 10 to 25% by weight of water.

9. Emulsion as claimed in any of claims 1 to 8, **characterized in that** it has a viscosity of 100 to 10,000 mPa.s at 23°C.

10. The use of the emulsion claimed in any of claims 1 to 9 as a body care preparation.

## Revendications

1. Emulsion d'eau dans l'huile ou d'huile dans l'eau renfermant :
(a) un polyol poly-12-hydroxystearate,
(b) un corps huileux choisi dans le groupe formé par les glycérides, les hydrocarbures, les huiles de silicone, les dialkyéthers et les dialkylcarbonates, ou leurs mélanges quelconques,
(c) 5 % à 25 % en poids d'eau,
**caractérisée en ce que**
le rapport pondéral des composants (a/b) est de 0,2 à 2,0.

2. Emulsion selon la revendication 1,
**caractérisée en ce que**
en tant que composant (b) elle renferme un dicapryléther, un glycéride en C₈-C₁₆ ou un mélange de ces substances.

3. Emulsion selon l'une des revendications 1 et 2,
**caractérisée en ce qu'**
elle renferme en outre au moins un agent d'humidification.

4. Emulsion selon l'une des revendications 1 à 3,
**caractérisée en ce qu'**
elle renferme en outre au moins un agent anti-irntation.

5. Emulsion selon l'une des revendications 1 à 4,
**caractérisée en ce qu'**
elle renferme en outre au moins un agent épaississant.

6. Emulsion selon l'une des revendications 1 à 5,
**caractérisée en ce qu'**
elle renferme en outre au moins un coémulsiant choisi dans le groupe des tensioactifs non ioniques.

7. Emulsion selon l'une des revendications 1 à 6,
**caractérisée en ce qu'**
en tant que coémulsifiant, elle renferme au moins un alkyl oligoglycoside.

8. Emulsion selon l'une des revendications 1 à 7,
**caractérisée en ce qu'**
elle renferme :
(a) 3 % à 25 % en poids de polyglycérolpoly-12-hydroxystéarate
(b) 20 % à 70 % en poids d'un corps huileux peu visqueux choisi dans le groupe formé par les glycérides en C₈-C₁₈, dicapryléthers ou les mélanges quelconques de ces substances
(c) 0 % à 25 % en poids d'alkyloligoglucosides
(d) 2 % à 10 % en poids d'au moins un agent humidifiant et
(e) 10 % à 25 % en poids d'eau.

9. Emulsion selon l'une des revendications 1 à 8,
**caractérisée en ce qu'**
elle présente une viscosité de 100-10000 mPa.s à 23°C.

10. Utilisation d'une émulsion selon l'une des revendications 1 à 9 en tant qu'agent de soins corporels.
